# EUROPEAN PATENT SPECIFICATION

(11) **EP 0 889 729 B1**
(45) Date of publication and mention of the grant of the patent: **05.06.2002**
(21) Application number: 97914328.6
(22) Date of filing: 17.03.1997
(51) Int. Cl.: A61K 35/78, A61P 1/10

(54) **LINSEED DRUG**
LEINSAMENMEDIKAMENT
MEDICAMENT A BASE DE GRAINES DE LIN

(30) Priority: 25.03.1996 FI 961364
(43) Date of publication of application: 13.01.1999
(73) Proprietor: Neomed OY, 53850 Lappeenranta (FI)
(72) Inventor: KOLARI, Pertti, FIN-54850 Kuukanniemi (FI); KOSKIMAA, Pentti, FIN-01830 Lepsämä (FI); GRÖHN, Pentti, FIN-00250 Helsinki (FI); KIVINEN, Anneli, FIN-17200 Vääksy (FI); TARPILA, Simo, FIN-00210 Helsinki (FI)
(74) Representative: Helino, Timo Kalervo
(86) International application number: FI9700174
(87) International publication number: WO9735594

(56) References cited:
- CH-A- 335 802
- DE-A- 3 110 510
- GB-A- 2 050 142
- GB-A- 18 941 282

## Description

The present invention relates to a drug composition as defined in the preamble of claim 1 and to a procedure for the manufacture of a drug composition as defined in the preamble of claim 7.

In the early 1970's, widespread interest in the fibrous material contained in food and its clinical importance was aroused. Based in the first place on epidemiological investigations, suggestions relating to the causal connections between many diseases and a low fibre content of food were presented and such diseases were termed fibre deficiency diseases.

Ailments of this type associated with the working of the alimentary canal include e.g. constipation, irritated-large-intestine syndrome, diverticulosis and haemorrhoids. Fibre deficiency is also considered to have a connection to hyperlipidaemia and disorders of sugar metabolism. It has also been suggested that there is a correlation between certain types of cancer in the intestinal area, e.g. cancer of the large intestine, and the amount of fibre contained in food.

'Food fibre' refers to that part of our vegetable diet which cannot be decomposed by the digestive enzymes and most of which is passed undigested through the alimentary canal.

Chemically, food fibres can be roughly divided into major subclasses, the most important of which are cellulose, hemicellulose and other polymeric carbohydrates as well as lignin, which is not a carbohydrate. Intestinal bacteria are able to decompose a considerable part of polymeric carbohydrates, but they are generally unable to decompose lignin.

As to their physiological properties, fibres can be divided into gelling or water soluble and non-gelling or water insoluble fibres.

Gelling fibres include e.g. pectin, guar gum and psyllium, which is obtained from the plantago ovata plant and generally used as fibre raw material in fibrous preparations registered as drugs. Apples, lemon fruits and oats preparations also contain plenty of gelling fibres.

Of the non-gelling fibres, among the best known is the bran obtained from the husks of cereal grain, e.g. wheat bran. Non-gelling fibre increases the bulk of faeces and normalizes the time of passage of food through the bowel; if bowel activity is slow (constipation), the presence of fibre will accelerate it, and if it is too fast (diarrhoea), fibre will retard it. Normal bowel activity is not affected by the presence of fibre. Gelling fibres absorb cholesterol in the bowel and increase its secretion into the faeces, thus reducing the cholesterol level of blood. Fibres are also described as having beneficial effects on the stabilization of the blood sugar balance.

'Bulk-laxative' refers to a purge that increases the contents and bulk of the bowel and is duly registered as a drug and used for certain indications approved for this preparation.

Bulk-laxative drugs are substantially different from natural products sold freely as foodstuffs, because the requirements relating to foodstuffs and those relating to drugs are completely different.

Among the commonest uses of bulk-laxatives are treatment of constipation, irritated-large-intestine syndrome and diverticulosis and its use for softening the faecal mass after surgical operations. Further, bulk-laxatives can be used as a form of auxiliary treatment in the case of hypercholesterolaemia and mild diabetes.

Prior-art bulk-laxatives are usually in the form of fine-grained powder. One of the best known bulk-laxatives is psyllium, which is obtained from the plantago ovata plant. A problem with prior-art preparations is that they generally swell quickly while still in the stomach, so their effect in the intestines is quite weak. Moreover, the amount of water they absorb is small.

As is known, linseed husks are a good fibre product for the regulation of bowel activity. They are relatively easy to husk, but separating the husk from the core after the husking is economically nearly impossible because of the identical size, weight and shape of the husk and the core. For this reason, linseeds are currently powdered without husking into an oleiferous mass which is finely pulverized and therefore also produces a quick effect in the stomach. Since the mass already acts in the stomach, it has only minor properties affecting bowel activity.

The oil content of linseeds is usually 35 - 40 %. Because of the oil, powdered linseed mass soon gets rancid, which is why various additives to be mixed with it have been developed, such as zinc and vitamin B, which prevent decomposition. In this case the fabrication and preservation should also be effected in protective gas.

The object of the present invention is to eliminate the above-mentioned drawbacks. A specific object of the invention is to present a new type of drug composition applicable for the treatment of a variety of disorders in bowel activity. Another object of the invention is to present a composition that is able to absorb plenty of water and does not swell until in the bowels. A further object of the invention is to present a product that keeps well without additives.

As for the features characteristic of the invention, reference is made to the claims.

In investigations relating to the invention, it was unexpectedly found that the linseed pulp left over when linseed oil is produced from linseed via cold pressing, if crushed into a suitable granular size, is well applicable for the treatment of functional disorders of the intestine. It was further established that the granular size of the crush has a significant effect on the water absorbency of the crush. Linseed crush powdered to a granular size as provided by the invention was found to have a good water absorbency and in addition the absorption of water took place over a long period of time.

The drug composition of the invention for the regulation of bowel activity, which composition contains linseed crush, is characterized in that the linseed crush is fabricated from linseeds by removing part of the oil contained in them via cold pressing, thus producing a relatively compact pulp. After this, the pulp is crushed and a crush fraction having a granular size of 2 - 5 mm is separated from it. In this way, insufficient granular sizes and powder, which would not keep well and which would produce an effect too quickly and therefore in the wrong place, are removed from the pulp.

In the procedure of the invention, inspected and controlled linseeds can be moved e.g. by means of a closed screw conveyor system to a press, e.g. a screw press, by means of which the seeds are pressed so that part of the oil contained in them is separated, thus producing linseed oil and linseed pulp, in the form of relatively hard pellets, measuring e.g. 1 cm x 1 cm, to be used as raw material of the drug composition of the invention. The compressed linseed pulp (pellets) is crushed e.g. in a roller mill. After this, the desired crush size is separated e.g. by screening, using e.g. a drum screen. By using stepless screen adjustment, the desired granular size can be defined very accurately.

The water absorbency of the linseed crush is generally at least 6 g, preferably at least 8 g / 1 g of dry matter of crush.

Maximal water absorption by the crush is generally reached in 3 - 10 hours, advantageously in 4 - 9 hours, preferably in 5 - 8 hours.

In an embodiment of the invention, the amount of linseed oil in the linseed crush is 5 - 30 %, preferably 10 - 20 %, e.g. 15 - 18 %. During cold pressing of linseed material, some linseed oil remains in the linseed pulp produced. By adjusting the pressure used in the cold pressing, it is possible to regulate the amount of oil left in the linseed pulp crush.

In an embodiment of the invention, the linseed crush is coated with a coat retarding the absorption of water. The coat may consist of e.g. a sweetening agent, e.g. sorbitol, lactose, saccharose, glucose, cyclamate or any other applicable coating material.

The granules of desired size according to the invention may be packed e.g. in plastic bottles provided with a gas-tight intermediate cap. The various stages of fabrication can be carried out under a protective gas to prevent oxidation of the linseed oil. The protective gas used may be e.g. nitrogen or any other protective gas applicable.

The invention has significant advantages over prior art. Since the seeds are pressed so as to remove part of the oil, a fairly hard and compact pulp is obtained that is easy to crush into a suitable granular size. Granules of suitable size form "capsules" in which most of the oil is contained and thus does not come into contact with air. Therefore, according to tests, the pulp can withstand even long times of storage (over 12 months) without getting rancid even if no preservatives or protective gases are used. In this way, a product is obtained that both keeps well and has a suitable granular size, in other words, a product that mainly produces an effect only after getting into the bowel.

Conventionally, the processing of seeds containing oil is performed at a high temperature, which has a detrimental effect both on the substances in the husks and on the fatty acid composition of the seed core. According to the present invention, the seeds are pressed without heating them (= cold pressing), so the oil contained in the seeds can be removed without producing detrimental effects on the fatty acid composition or the substances in the husks of the seeds.

The linseed crush of the invention has a better water absorbency than most other corresponding preparations available on the market. Further, the absorption of water takes place slowly, which means that the crush will only absorb water and swell after getting into the bowel, as it is intended to, and not already in the stomach, as fine-grained powders available on the market do. Some of the intended effects of a bulk-laxative cannot be attained if it swells completely already in the stomach.

In addition to the insoluble fibre in linseed crush acting as a laxative that increases the bowel contents and is applicable for the treatment of constipation, the soluble fibre has the effect of absorbing cholic acids, thus reducing the blood cholesterol level and stabilizing the blood sugar balance. Unsaturated fatty acids (omega-3 and omega-6) and vegetable sterols, which are abundantly present in the oil remaining in the crush, further enhance the cholesterol reducing effect of the linseed fibre oil drug.

Further, the phospholipids contained in the oil in the crush protect the mucous membrane of the stomach against the harmful effects of acids, analgesic drugs and helicobacteria.

In addition, linseed crush is agreeable to the user because it has a pleasant, somewhat nut-like taste and the drug dose can be taken in a variety of ways, e.g. scattered on curdled milk, porridge, yoghurt or other food or mixed in juice or other liquid.

In the following, the invention is described in detail by the aid of examples, which are only intended to illustrate the invention without restricting it.

### EXAMPLE 1

The water absorbency and swelling of linseed crush were tested by soaking linseed crush powdered to different granular sizes in a graduated glass. The tested crush consisted of linseed crush fabricated according to the invention and having a granular size of 2 - 5 mm. Commercially available bulk-laxatives are usually of a granular size below 2 mm. In the test, 1 g of both crush types was weighed and placed in a graduated glass, into which was added 50 ml of water. The crush was mixed in water by shaking gently, whereupon the swelling was observed by reading the crush volume from the scale on the graduated glass. Table 1 shows the test results.

**Table 1.**

| Swelling of linseed crush (volume/ml) during sedimentation. | | | | | | | | | | |
|---|---|---|---|---|---|---|---|---|---|---|
| | Time | | | | | | | | | |
| Granular size | 0 h | 1 h | 2 h | 3 h | 4 h | 5 h | 6 h | 7 h | 8 h | 24 h |
| < 2 mm | 2.5 | 8 | 9 | 9 | 9 | 9 | 9 | 9 | 9 | 9 |
| 2 - 5 mm | 3 | 8 | 9 | 10 | 14 | 14 | 14 | 14 | 14 | 14 |

As can be seen from Table 1, the granular size of the crush has a pronounced effect on the water absorbency of the crush. Crush of a larger granular size is able to absorb more water and the absorption takes place more slowly than in finer-grained crush. It is desirable that the crush should have a high water absorption capacity and that the absorption should take place slowly, because this ensures that the swelling of the crush will not occur already in the stomach but only after it has got into the bowels.

### EXAMPLE 2

The properties of the linseed crush of the invention were analyzed by determining its water-soluble and water-insoluble food fibre, total pentosans, water-soluble pentosans, beta-glucan, total phosphatides, lecithin fraction (phosphatidyl choline) and water absorbency. The proportions of water-soluble and water insoluble food fibre were determined by Asp's enzymatic-gravimetric method (Journal of Agricultural and Food Chemistry 31 (1983) 476-482). The enzymes used in the method were alpha amylase, pepsin and pancreatin. The pentosans were determined by the Douglas spectrophotometric method. Beta-glucan was determined by the McCleary beta-glucanase method using the Megazyme reagent package. The phosphatides were determined by extracting the residual oil of the crush by means of a Soxhlet extractor with an azeotropic chloroform-methanol mixture. By this method, the residual oil content of the crush was found to be 17 %. From the oil obtained, the total phosphatides were determined via a total phosphor analysis using the coefficient 30 and a method according to the American Oil Chemists' Society AOCS Official Method Ca 12-15, 1988. The lecithin was determined as a substance insoluble in acetone from the residual oil obtained from the Soxhlet extraction, according to the method presented in the publication FAO Food & Nutrition Paper 17, 1980. The water absorbency of the crush was determined by mixing a 2.5 g sample in water. The mixture was allowed to stand for 30 minutes at a temperature of 30 °C, shaken at 5-minute intervals. After the sedimentation, the mixture was centrifuged, the clear solution was poured away and the sediment remaining in the centrifuge tube was weighed. The result was calculated for the dry matter of the crush (92.1 %). The analysis results are presented in Table 2.

**Table 2.**

| Properties of linseed crush | |
|---|---|
| Water-soluble food fibre | 11.0 g /100 g of crush |
| Water-insoluble food fibre | 33.2 g / 100 g of crush |
| Total pentosans | 8.1 g / 100 g of crush |
| Water-soluble pentosans | 1.2 g / 100 g of crush |
| Beta-glucan | 0.38 g / 100 g of crush |
| Total phosphatides | 7.4 g / 100 g of residual oil, corresponding to 1.3 g / 100 g of crush |
| Lecithin | 4.6 g / 100 g of residual oil |
| Water absorbency | 8.3 g water / 1 g of dry matter of crush |

## Claims

1. Drug composition for the regulation of bowel activity, said composition containing linseed crush, **characterized in that** the linseed crush has been fabricated by removing part of the oil contained in linseeds via cold pressing, crushing the substantially compact linseed pulp cake or pellets obtained by pressing and separating from it the portion of crush that has a granular size of 2 - 5 mm.

2. Composition as defined in claim 1, **characterized in that** the crush has a water absorbency of at least 6 g, preferably at least 8 g of water / 1 g of dry matter.

3. Composition as defined in claim 1 or 2, **characterized in that** maximal water absorption by the crush is attained in 3 - 10 hours, advantageously in 4 - 9 hours, preferably in 5 - 8 hours.

4. Composition as defined in any one of claims 1 - 3, **characterized in that** the amount of linseed oil contained in the linseed crush is 5 - 30 %, preferably 10 - 20 %, e.g. 15 - 18 %.

5. Composition as defined in any one of claims 1 - 4, **characterized in that** the linseeds are cold-pressed using a screw press.

6. Composition as defined in any one of claims 1 - 5, **characterized in that** the linseed crush is coated with a coat retarding the absorption of water.

7. Procedure for the fabrication of a drug composition for the regulation of bowel activity as defined in any one of claims 1 - 6, said composition containing linseed crush, **characterized in that** part of the oil contained in the linseeds is removed by cold-pressing so that they form a substantially compact pulp cake, the pulp cake thus obtained is crushed to a granular size below 5 mm and from the crushed pulp is removed the portion that has a granular size under 2 mm.

8. Procedure as defined in claim 7, **characterized in that** the linseeds are cold-pressed using a screw press.

9. Procedure as defined in claim 7 or 8, **characterized in that** the linseed pulp is crushed in a roller mill.

10. Procedure as defined in any one of claims 7 - 9, **characterized in that** the crush is separated by screening.

11. Procedure as defined in any one of claims 7 - 10, **characterized in that** a protective gas is used in the procedure.

12. Procedure as defined in any one of claims 7 - 11, **characterized in that** the linseed crush is coated with a coat retarding the absorption of water.

13. Procedure as defined in any one of claims 7 - 12, **characterized in that** the linseed crush is packed in air-tight packages.

## Patentansprüche

1. Medikamentenzusammensetzung für die Regulierung der Darmtätigkeit, welche Zusammensetzung Leinsamenschrot enthält, **dadurch gekennzeichnet, daß** der Leinsamenschrot bearbeitet wurde durch Entfernen eines Teils des im Leinsamen enthaltenen Öls durch Kaltpressung, Schroten des im wesentlichen kompakten Leinsamenmassekuchens oder von Pellets, die durch Pressen erhalten wurden, und Abtrennen des Anteils von Schrot, der eine Korngröße von 2-5mm hat, hiervon.

2. Zusammensetzung nach Anspruch 1, **dadurch gekennzeichnet, daß** der Schrot eine Wasseraufnahmefähigkeit von wenigstens 6 g, vorzugsweise wenigstens 8 g Wasser / 1 g Trockenmasse hat.

3. Zusammensetzung nach Anspruch 1 oder 2, **dadurch gekennzeichnet, daß** die maximale Wasseraufnahme durch den Schrot nach drei bis zehn Stunden, vorteilhaft nach vier bis neun Stunden, bevorzugt nach fünf bis acht Stunden erhalten ist.

4. Zusammensetzung nach einem der Ansprüche 1 bis 3, **dadurch gekennzeichnet, daß** die Menge des in dem Leinsamenschrot enthaltenen Leinöls 5 - 30 %, vorzugsweise 10 - 20 %, z.B. 15 - 18 % beträgt.

5. Zusammensetzung nach einem der Ansprüche 1 bis 4, **dadurch gekennzeichnet, daß** der Leinsamen unter Verwendung einer Schraubenpresse kalt gepreßt ist.

6. Zusammensetzung nach einem der Ansprüche 1 bis 5, **dadurch gekennzeichnet, daß** der Leinsamenschrot mit einer Beschichtung, welche die Aufnahme von Wasser verzögert, versehen ist.

7. Verfahren für die Herstellung einer Medikamentenzusammensetzung für die Regulierung der Darmtätigkeit nach einem der Ansprüche 1 bis 6, wobei die Zusammensetzung Leinsamenschrot enthält, **dadurch gekennzeichnet, daß** ein Teil des in dem Leinsamen enthaltenen Öls durch Kaltpressen entfernt wird, so daß er einen im wesentlichen kompakten Massekuchen bildet, der so erhaltene Massekuchen auf eine Korngröße von weniger als 5 mm geschrotet wird und aus der geschroteten Masse der Anteil, welcher eine Korngröße von weniger als 2 mm hat, entfernt wird.

8. Verfahren nach Anspruch 7, **dadurch gekennzeichnet, daß** der Leinsamen unter Verwendung einer Schraubenpresse kalt gepreßt wird.

9. Verfahren nach Anspruch 7 oder 8, **dadurch gekennzeichnet, daß** die Leinsamenmasse in einer Walzenmühle geschrotet wird.

10. Verfahren nach einem der Ansprüche 7 bis 9, **dadurch gekennzeichnet, daß** der Schrot durch Siebung getrennt wird.

11. Verfahren nach einem der Ansprüche 7 bis 10, **dadurch gekennzeichnet, daß** bei dem Verfahren ein Schutzgas verwendet wird.

12. Verfahren nach einem der Ansprüche 7 bis 11, **dadurch gekennzeichnet, daß** der Leinsamenschrot mit einer Schicht beschichtet wird, welche die Aufnahme von Wasser verzögert.

13. Verfahren nach einem der Ansprüche 7 bis 12, **dadurch gekennzeichnet, daß** der Leinsamenschrot in luftdichten Packungen verpackt wird.

## Revendications

1. Composition médicamenteuse pour la régulation de l'activité de l'intestin, ladite composition contenant du jus de graines de lin, **caractérisée en ce que** le jus de graines de lin a été fabriqué en enlevant une partie de l'huile contenue dans les graines de lin au moyen d'une pression à froid, en écrasant le gâteau ou les boulettes de graines de lin substantiellement compacts obtenus en pressant et en séparant la portion de jus ayant une taille granulaire de 2-5 mm.

2. Composition selon la revendication 1, **caractérisée en ce que** le jus a une absorbance d'eau d'au moins 6g, de préférence au moins 8g d'eau/1 g de matière sèche

3. Composition selon la revendication 1 ou 2, **caractérisée en ce que** l'absorption maximale de l'eau par le jus est atteinte en 3-10 heures, et avantageusement en 4-9 heures, de préférence en 5-8 heures.

4. Cnmposition selon l'une quelconque des revendications 1 à 3, **caractérisée en ce que** la quantité d'huile de lin contenue dans le jus de graines de lin est de 5-30%, de préférence 10-20%, par exemple 15-18%.

5. Composition selon l'une quelconque des revendications 1-4, **caractérisée en ce que** les graines de lin sont pressées à froid en utilisant une presse à vis.

6. Composition selon l'une quelconque des revendications 1-5, **caractérisée en ce que** le jus de graines de lin est revêtu d'un revêtement retardant l'absorption d'eau.

7. Procédure pour la fabrication d'une composition médicamenteuse pour la régulation de l'activité de l'intestin comme définie dans Tune quelconque des revendications 1-6, ladite composition contenant du jus de graines de lin, **caractérisée en ce que** une partie de l'huile contenue dans les graines de lin est enlevée par une pression à froid de façon à obtenir un gâteau de pulpe compact, le gâteau de pulpe ainsi obtenu est écrasé en une taille granulaire inférieure à 5 mm et de cette pulpe écrasée, on enlève la portion qui a une taille granulaire inférieure à 2 mm.

8. Procédure selon la revendication 7, **caractérisée en ce que** les graines de lin sont pressées à froid en utilisant une presse à vis.

9. Procédure selon la revendication 7 ou 8, **caractérisée en ce que** la pulpe de grains de lins est écrasée dans un broyeur à rouleaux.

10. Procédure selon l'une quelconque des revendications 7-9, **caractérisée en ce que** le jus est séparé par tamisage.

11. Procédure selon l'une quelconque des revendications 7-10, **caractérisée en ce qu'**un gaz protecteur est utilisé dans la procédure.

12. Procédure selon l'une quelconque des revendications 7-11, **caractérisée en ce que** le jus de graines de lin est revêtu d'un revêtement retardant l'absorption de l'eau.

13. Procédure selon l'une quelconque des revendications 7-12, **caractérisée en ce que** le jus de graines de lin est emballé dans des emballages étanches à l'air.
